# EUROPEAN PATENT APPLICATION

(11) **EP 1 955 589 A1**
(43) Date of publication of application: **13.08.2008**
(21) Application number: 06834157.7
(22) Date of filing: 30.11.2006
(51) Int. Cl.: A01H 5/00, C12N 15/09, C12N 15/82, C12N 5/10

(54) **METHOD FOR TRANSFORMATION OF PLANT BELONGING TO FAMILY ARACEAE**

(30) Priority: 01.12.2005 JP 2005348259
(71) Applicant: Kirin Holdings Kabushiki Kaisha, Tokyo (JP)
(72) Inventor: UMEMOTO, Naoyuki, Sakura-shi, Tochigi 3291414 (JP); MAMIYA, Kanji, Sakura-shi, Tochigi 3291414 (JP)
(74) Representative: HOFFMANN EITLE
(86) International application number: PCT/JP2006/324402
(87) International publication number: WO 2007/064037

(57) **Abstract**

The present invention provides a method for transforming a plant of the family Araceae, comprising introducing a foreign gene into an embryogenic callus of the plant through infection of the callus with Agrobacterium or a microorganism capable of introducing a gene therein by an analogous mechanism to that of Agrobacterium, thereby preparing a transformed embryogenic callus.

SEQUENCE LISTING

<110> Kirin Holdings Kabushiki Kaisha

<120> Method for transforming plant of the family Araceae

<130> PH-2974-PCT

<140> PCT/JP2006/324402
<141> 2006-11-30

<150> JP 2005-348259
<151> 2005-12-01

<160> 2

<170> PatentIn Ver. 2.1

<210> 1
<211> 20
<212> DNA
<213> Artificial Sequence

<220>
<223> Description of Artificial Sequence:primer

<400> 1
agagatacgc cctggttcct                                                20

<210> 2
<211> 20
<212> DNA
<213> Artificial Sequence

<220>
<223> Description of Artificial Sequence:primer

<400> 2
ataaataacg cgcccaacac                                                20

## Description

### TECHNICAL FIELD

The present invention relates to a method for introducing a gene into a plant of the family Araceae, and to a method for transforming a plant of the family Araceae with a gene.

### BACKGROUND OF THE INVENTION

Known plants of the family Araceae include important food crops such as aroid (*Colocasia esculenta*), tropical Colocasia, and *Amorphophallus konjac,* popular and important foliage plants such as Spathiphyllum, Anthurium, Caladium, Aglaonema, Alocasia, Dieffenbachia, Monstera, Philodendron, Pothos (*Epipremnum aureum),* and Syngonium, and medicinal plants such as Pinellia and *Acorus gramineus.*

Of these plants, about 30 species of plants of the genus *Spathiphyllum* are distributed in tropical America and 2 species of the genus are distributed in Southeast Malaysia. Approximately 10 of the species are cultivated as pot foliage plants and also used for ground cover in the tropical and subtropical zones. These plants are the most popular foliage plants. These plants also have shade tolerance and come into flower indoors, so that there are high expectations for them as plants for developing favorable indoor environments in addition to exerting of "soothing" effects when they are viewed indoors.

In recent years, with the progress of plant biotechnology, cultivars having various properties are bred using techniques such as tissue culture, anther culture, cell culture, cell fusion, and mutation treatment. Furthermore, owing to development of gene (DNA) recombination techniques, a gene of an organism species is introduced into another organism species that is generally unable to be crossed with the first species, so that a cultivar having conventionally unpredictable properties are bred. Cultivation of such genetic recombinant plants is widely conducted such that their cultivated acreage is increased in America, Canada, Argentina, and China. To breed a cultivar using genetic recombination, isolation of a gene having required functions, and gene transfer competent with a subject plant, are necessary. As a method for introducing a foreign gene into a plant, a method using Agrobacterium, an electroporation method, a particle gun method, a PEG method, a whisker method, and the like are known. Vectors for introducing a foreign gene into plant cells and various reporter or marker genes for selection and recognition of transformed cells, as well as regulatory genes (e.g., a promoter) for expression of the introduced foreign gene, have been developed with repeated trial and error. However, it has generally been thought that transformation of plants of the family Araceae which includes Spathiphyllum, is difficult even if the above methods or means are employed. Successful examples are known for only few types of such plants.

For example, JP Patent Publication No. 10-75673 A (1998) discloses a combined system of a plant regeneration from protocorm-like multiple shoots derived from the calli of Pinellia medicinal plants of the family Araceae, with a gene transfer using particle gun. This document, however, does not disclose the number of plants obtained or an efficiency using the system. The level of difficulty for implementation of this method is very high, so that only few experienced researchers might be able to implement this method. Furthermore, in this document, it is concluded that the transformation of monocotyledons, including plants of the family Araceae, with Agrobacterium is difficult, since the plants are not infected with Agrobacterium.

Similarly, Fukino et al. (Fukino, N, Hanada, K., Ajisaka, H., Sakai, J., Hirochika, H., Hirai, M., Hagio, T., Enomoto, S. (2000) Jpn Agri. Res. Quarterly, 34 (3), 159-165) clearly concludes that the transformation of monocotyledons, including Colocasia of the family Araceae, with Agrobacterium is difficult, since the plant is not infected with Agrobacterium. They also report that transformants were obtained by using a particle gun for calli (Karube, M., Shimonishi, K., Kukimura, H. (1992) Jpn. J. Breed., 42 (1), 58-59) derived from tubers of *Colocasia esculenta* cv. 'Eguimo'. However, as described by Fukino et al. (*supra*), the experiment using 2 g of callus was repeated 96 times to obtain only two transformants, showing an extremely low efficiency.

Among plants of the family Araceae, Anthurium transformants have been reported by a group of the University of Hawaii. A transformation method established by the group is disclosed in Kuehnle, A. R., Chen F.-C., Sugii N. C. (2001) Biotechnology in Agriculture and Forestry, 48, 3-15. Specific data obtained from experimentation are disclosed in Chen F.-C., Kuehnle A. R. (1996) J. Amer. Soc. Hort. Sci., 121 (1), 47-51; and Kuehnle, A.R., Fujii, T., Chen, F.-C., Alvarez, A., Sugii, N., Fukui, R. , Aragon, S.L., Jaynes. J.M. (2004) HortSci., 39 (6), 1327-1331). The materials that they considered to be best are blanched internodes, which are infected with Agrobacterium to induce calli within 1-2 months. Shoots, which have slightly greened within 1 month and completely greened within 9 months under week light, are induced, and then rooted within 1-2 months, thereby leading to small plants. It is also described that 2 years are required before flowering. It is described that the gene transfer efficiency was 1.3% when root was used as a material for transformation. The gene transfer efficiency achieved when blanched internodes are used as the materials, however, are unknown. Although Chen et al. (*supra*) described that several hundred drug-resistant plants were obtained; however, the actual introduction of a gene was confirmed for only 6 lines by PCR or the like, the mRNA expression was confirmed for 2 lines, and the protein expression was confirmed for only one (1) line. Kuehnle et al. (2004; *supra)* reported that they obtained 13 lines of transformants and that it took 21 months in total for obtaining them, suggesting that long-term culture is required for generating the transformants. Furthermore, the chimeric rate was as high as 26-62%, indicating the need for another selection method (Kuehnle et al. (2001; *supra*).

Thus, to realize an efficient transformation of plants of the family Araceae, it is required to perform more efficient transformation into plant cultures and to select and produce culture materials whose transformation is easy.

In case of Spathiphyllum, somatic embryos have ever been obtained from filaments for transformation (Werbrouck, S.P.O., Eeckhaut, T.G.R, Debergh, P.C. (2000) Acta Horticulturae, 520, 263-269); however, the technique employed does not involve induction and proliferation of embryogenic calli but a method of inducing somatic embryos directly from filaments. Hence, the proliferation rate achieved by use of one explant is extremely restrictive (although no proliferation rate is described therein). Furthermore, somatic embryos are induced without being separated from filaments, suggesting that such conditions are unfavorable for subsequent regeneration of plants. In addition, the method is unsuitable in that where filaments are used as explants, then the time for collecting filaments is limited to the flowering time and the number of initial filament materials is limited for the purpose of proliferation. Actually, they have not reported any successful case concerning transformation of any material including said filament materials.

The present inventors have examined sites suitable for induction of an embryogenic callus with the use of various tissues of Spathiphyllum culture seedlings that are maintained as stock plants. As a result, the present inventors have now found that a leaf sheath, especially its base or a part near the base, exerts the highest callus induction rate and that embryogenic calli exist at high frequency among these calli. The present inventors further have now succeeded in developing a dramatically efficient method for genetic recombination by mediating Agrobacterium or a microorganism capable of introducing a gene by an analogous mechanism to that of Agrobacterium, by using a callus (i.e., an embryogenic callus) as a culture material with extremely high efficiency for inducing somatic embryos from leaf sheaths.

### DISCLOSURE OF THE INVENTION

An object of the present invention is to provide a highly efficient method for introducing a gene into plants of the family Araceae, which are expected to serve as plants for foods, foliage plants, medicinal plants, or plants to be subjected to genetic engineering, and to provide a method for producing such transformed plants.

The present inventors have conducted concentrated studies and experiments to achieve the above object. As a result, the present inventors have now found that the efficiency of gene introduction into plants of the family Araceae and the production efficiency of the transformed plants can be significantly improved by: obtaining embryogenic calli from leaf sheaths having a good ability to redifferentiate into plants, particularly from the base of the leaf sheath or a part near the base; infecting the calli with Agrobacterium (i.e., a microorganism belonging to the genus *Agrobacterium)* or a microorganism capable of introducing a gene by an analogous mechanism to that of Agrobacterium; performing a coculture of them; selecting transformed embryogenic calli using an antibiotic; and regenarating the thus obtained transformed embryogenic calli so as to induce seedling plants from somatic embryos. Thus, the present inventors have completed the present invention.

Thus, the present invention is summarized as having the following characteristics.

In an aspect, the present invention provides a method for transforming a plant of the family Araceae, which comprises introducing a foreign gene into an embryogenic callus of the plant through infection of the callus with Agrobacterium or a microorganism capable of introducing a gene by an analogous mechanism to that of Agrobacterium, thereby producing a transformed embryogenic callus.

In one embodiment, the embryogenic callus is from a leaf sheath as explant.

As used herein, the term "leaf sheath" refers to a leaf base which surrounds the stem in the form of a sheath.

In another embodiment, the plant of the family Araceae is a plant of the genus *Spathiphyllum.*

In another embodiment, the method of the present invention further comprises inducing an somatic embryo from the transformed embryogenic callus, followed by germination and rooting, thereby producing a transformed plant.

The term "somatic embryo" as used herein refers to an embryo generated from a cultured plant cell (or tissue), which is capable of developing into a normal plant.

The present invention enables transformation of plants of the family Araceae, which has been thought to be difficult, with Agrobacterium or a microorganism capable of introducing a gene by an analogous mechanism to that of Agrobacterium. So, by the present invention, it becomes easy to introduce a foreign gene into plants. The present invention provides such a significant advantage that a transformed plant of the family Araceae can efficiently be produced accordingly.

This description includes all or part of the contents as disclosed in the description and/or drawings of Japanese Patent Application No. 2005-348259, which is a priority document of the present application.

### BRIEF DESCRIPTION OF THE DRAWINGS

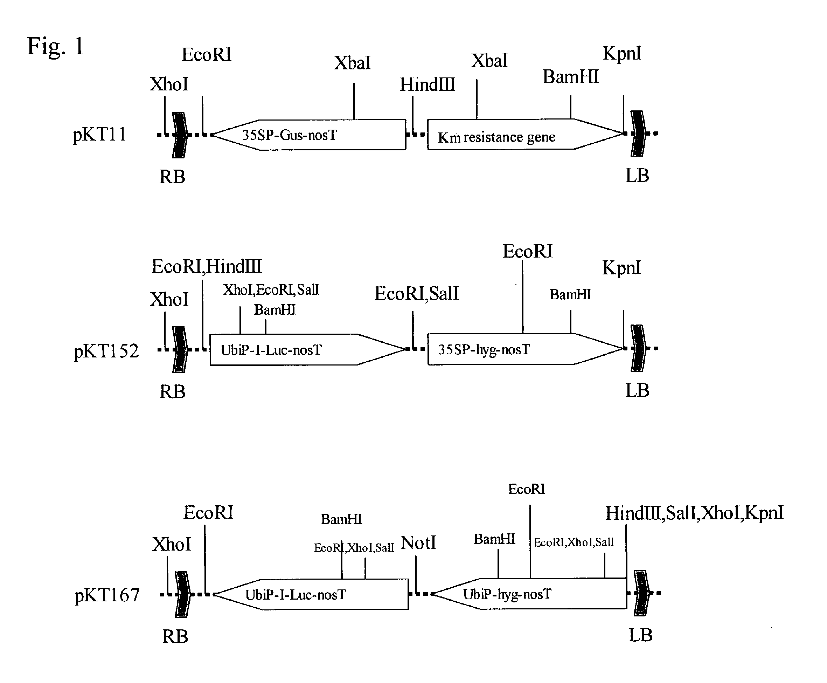

Fig. 1 shows vector structures used for transformation. In Fig. 1, pKT11 denotes a basic vector, and pKT152 and pKT167 denote the plant transformation vectors of the present invention, respectively, wherein RB denotes a right border sequence, LB denotes a left border sequence, 35SP-Gus-nosT denotes a β glucuronidase gene that is driven by a cauliflower mosaic virus 35S promoter, Km resistance gene denotes a kanamycin resistance gene, UbiP-I-Luc-nosT denotes an intron-containing luciferase gene that is driven by a Maize ubiquitin promoter, 35SP-hyg-nosT denotes a hygromycin resistance gene that is driven by a cauliflower mosaic virus 35S promoter, and UbiP-hyg-nosT denotes a hygromycin resistance gene that is driven by a Maize ubiquitin promoter.

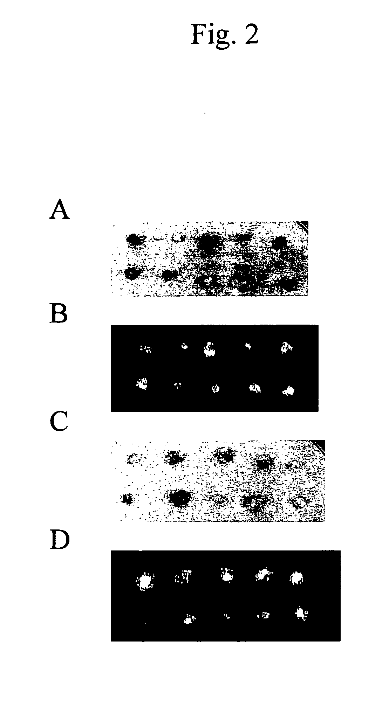

Fig. 2 shows embryogenic calli on day 7 after they were transferred onto the callus selection medium, and luciferase luminescence activity. Specifically, Fig. 2 shows the results of measuring the luminescence of an embryogenic callus mass on day 7 after its treatment with an Agrobacterium carrying each vector, at the bright field (pKT152:A, pKT165:C) or at the dark field (ARGUS50 measurement level: 0 to 3, pKT152:B, pKT165:D). In the measurement of luminescence, the images were processed so that the larger the amount of luminescence the brighter the image. Ten (10) embryogenic callus masses were subjected to each measurement. The results reveal that the embryogenic calli were efficiently infected with agrobacteria and that the luciferase gene was expressed in the embryogenic calli after introduction of said gene.

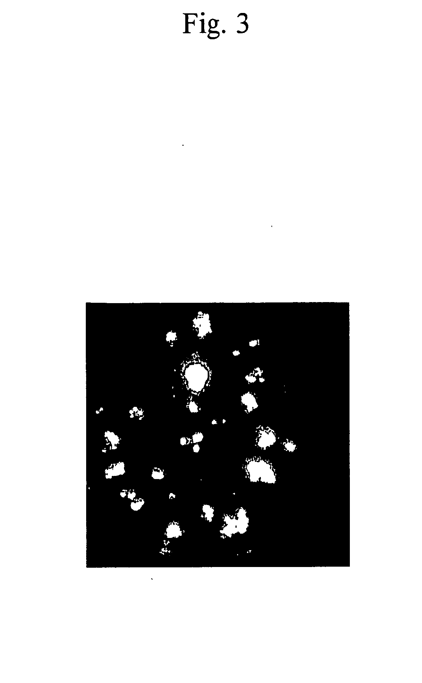

Fig. 3 shows transformed embryogenic calli on month 3 after they were transferred onto the callus selection medium, and luciferase luminescence activity. Specifically, Fig. 3 shows the results of measuring the luminescence at the dark field (ARGUS50 measurement level: 0 to 5) of an embryogenic callus mass that was treated with an Agrobacterium carrying vector pKT165 and then subjected to 3-months culture (after subcultured every month). In the measurement of luminescence, the images were processed so that the larger the amount of luminescence the brighter the image. The photograph shows the result of one of 2 experiments. Among total 42 embryogenic callus masses that were transferred in the 2 experiments, 22 masses and 5 masses were confirmed as strongly emitting and as weekly emitting, respectively. The strongly emitting embryogenic calli were non-chimeric and uniform transformants.

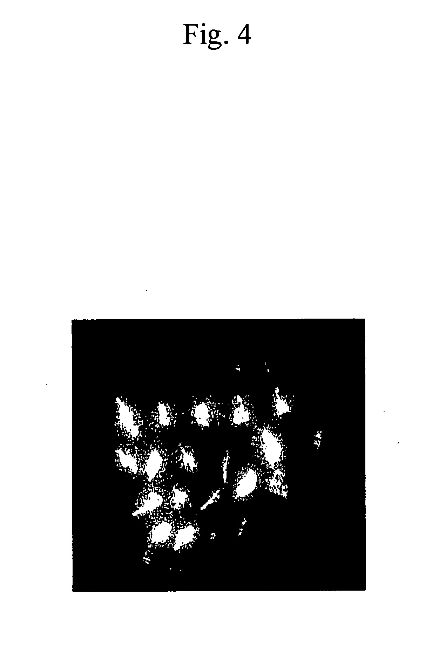

Fig. 4 shows luciferase luminescence activity in leaf discs of regenarated plants.
Specifically, Fig. 4 shows the results of measuring the luminescence at the dark field (ARGUS50 measurement level: 0 to 5) of the leaf discs of the regenerated plants, in which the gene transfer was confirmed by PCR, among the regenerated plants. In the measurement of luminescence, the images were processed so that the larger the amount of luminescence the brighter the image. Although some leaf discs did not emit due to absorption of a reagent or emitted unevenly, the luminescence on almost all surfaces of sixteen (16) emitting leaf discs could be observed, indicating a very low chimeric property.

### PREFERRED EMBODIMENTS OF THE INVENTION

### (1) Host and plant site

Examples of plant hosts for transformation include plant cells of the family Araceae, and particularly preferable examples of the same include plant cells of aroid *(Colocasia esculenta),* Colocasia, *Amorphophallus konjac,* Spathiphyllum, Anthurium, Caladium, Aglaonema, Alocasia, Dieffenbachia, Monstera, Philodendron, Pothos (*Epipremnum aureum*), Syngonium, calla, white arum (*Lysichiton camtschatcense*), skunk cabbage (*Symplocarpus foetidus*), and sweet grass (*Acorus calamus*). Cultivars of the most preferable Spathiphyllum thereof are not particularly limited and commercially available cultivars thereof (e.g., Petite or Double Take and Claudia of Twyford, U.S.A.) can be used.

Examples of plant materials include leaf sheaths, shoot apex, shoot primordiums, meristems, leaf discs, stem sections, root sections, tuber sections, petiole sections, protoplasts, calli, anthers, pollens, pollen tubes, flower stalk sections, scape sections, petals, and sepals. Preferable examples are parts near the bases of these materials, more preferably a leaf sheath base or a part near the leaf sheath base. As used herein, the term "leaf sheath base" refers to a 1/4 lower part of the leaf sheath and the term "a part near the leaf sheath base" refers to a 1/2 lower part of the leaf sheath excluding the leaf sheath base.

### (2) Induction of embryogenic callus

In the present invention, the embryogenic callus is capable of regenarating into a plant, is in a soft form (easily disintegrated by applying a force), has a diameter of less than 1 mm, and is composed of cream-colored particular cells or tissues that have regenarated. Any embryogenic callus may be used in the present invention, as long as it is prepared by culturing tissue sections of the family Araceae, is capable of regenarating into the plant level after introduction of a gene, and can be infected with Agrobacterium. Preferablly, the embryogenic calli are prepared by transferring *in vitro* tissue sections onto a medium for callus induction and then culturing them to induce calli.

It is not always required to collect a leaf sheath, which is a preferable example of material, from culture seedlings. Leaf sheaths may also be collected from plants that are maintained under open conditions such as a greenhouse. In such case, leaf sheaths are subjected to surface sterilization according to a conventional method, so as to prepare explants. When culture seedlings are used, the surface sterilization is not required. Any sites may also be used as long as they are of leaf sheaths. Parts near the leaf sheath base are preferred. More preferably, when bases, for example leaf sheaths, are collected from plant seedlings, leaf sheaths are collected by peeling off the sheaths from the plant seedlings without cutting with a scalpel or the like. Sites including the peeled portions are used. Although calli may also be obtained from leaves or roots, the use of leaves or roots is unfavorable because of its significantly low obtainment frequency compared with the case of using leaf sheaths, or because of difficulty in embryogenic callus induction in the subsequent steps.

Next, the method that comprises transferring the prepared leaf sheath explants onto a medium to produce embryogenic calli will be described below.

A leaf sheath explant obtained as described above is transferred onto a callus induction medium, to induce calli. MS medium (Physiol. Plant., 15, p. 143, 1962) or the like, which is generally used for tissue culture, is used as the basic medium for callus induction. 1-6%, desirably 2-4%, sucrose (weight/volume%, which will also be applied to % below) is used as a sugar source. As an example of desirable auxins that are plant growth regulatory substances, 1-8 ppm, desirably 3-5 ppm, 2,4-dichlorophenoxyacetic acid (2,4-D) is used. As examples of the other auxins, indole-3-acetic acid (IAA), indole-3-butylic acid (IBA), 1-naphthalenacetic acid (NAA), 4-chlorophenoxyacetic acid (CPA), chloromethylphenoxyacetic acid (MCPA), 2,4,5-trichlorophenoxyacetic acid (2,4,5-T), dichloromethoxybenzoic acid (DICAMBA), and trichloroaminopicolinic acid (PICROLAM) are used. As a desirable example of cytokinins, 0.05-2 ppm, desirably 0.1-0.3 ppm, 6-benzyladenine (BA) is used. Used as the other cytokinins are zeatin (ZEA), kinetin (KN), 6-(benzylamino)-9-(2-tetrahydropyranyl)-9H-purine (PBA), 2-isopentenyladenine (2ip), thidiazuron (TDZ), and the like. The best results can be obtained under the condition that 2,4-D and BA are used in combination. When 2,4-D is less than 1 ppm, a plant is easily generated together with calli. When 2,4-D is higher than 8 ppm, the tissue will be easily browned. Moreover, 50-200 ppm caseinate hydrolysate and 0.1-10 mM MES as buffer may also be added. The pH of the medium is adjusted to 5 to 7. After pH-adjustment, agar (0.8-1.2%) or Gel-Rite (0.1-0.3%) is used to solidify the medium. A container to be used herein is not particularly limited, as long as it is used for plant tissue culture (e.g., a plant box, Asahi Techno Glass (Tokyo, Japan); inner volume: 300 ml). Regarding light environment, a dark place is employed. The temperature ranges from 20°C to 30°C and desirably from 23°C to 27°C. The culture period ranges from 4 to 12 weeks and desirably from 5 to 8 weeks.

The thus induced calli are subcultured on the same medium. Embryogenic calli that have been generated are selected and then transplanted onto the same medium, so that embryogenic calli alone proliferate. Subsequently, embryogenic calli are maintained and caused to proliferate with similar procedures. Light and temperature conditions that are the same as those for callus induction are employed. The culture period ranges from 4 to 6 weeks.

Specifically, if once such embryogenic calli can be obtained, embryonic callus induction is not required every time (for each experiment) for a transformation material. It becomes possible to grow only a necessary amount of embryogenic callus to use the callus for a transformation experiment.

### (3) Agrobacterium infection method

In the method of the present invention, the introduction of a foreign gene comprises immersing embryogenic calli in a solution for infection with Agrobacterium, which carries a gene of interest, or a microorganism capable of introducing a gene therein by an analogous mechanism to that of the Agrobacterium, and then performing coculture of the immersed tissue portion in a coculture medium. In recent years, it has been reported that the gene transfer takes place in plants with the genus *Rhizobium* (other than Agrobactereium) by a mechanism analogous to that of Agrobacterium (Chilton, M.D. (2005) Nat. Biotechnol., 23 (3), 309-10). Hence, the efficiency of introduction achieved is equivalent to that achieved by use of Agrobacterium so that the use of both microorganisms have been aggressively attempted (Weir, B. J., Mitchell, H. J., Broothaerts, W., Jefferson, R. A. (2005) Plant Biology 2005 Final Program, 93). As is also clear from this, for gene introduction, not only Agrobacterium, but also microorganisms (e.g., microorganisms of the genus *Rhizobium,* the genus *Sinorhizobium,* and the genus *Mesorhizobium)* capable of introducing a gene therein by their mechanisms analogous to that of Agrobacterium can also be used. Further, note that *Agrobacterium tumefaciens* is registered as the genus *Rhizobium* with American Type Culture Collection (ATCC) (U.S.A.).

As Agrobacterium, *Agrobacterium tumefaciens* is preferred and specifically the AGL0 strain, the LBA4404 strain, the CIB542/A136 strain, the EHA101 strain, and the like can be used, but the examples are not limited thereto.

As used herein, the phrase "capable of introducing a gene by an analogous mechanism to that of Agrobacterium" means transformation with not only Agrobacterium, but also with microorganisms (e.g., microorganisms of the genus *Rhizobium,* the genus *Sinorhizobium,* and the genus *Mesorhizobium*) by mechanisms similar to that of Agrobacterium.

Foreign genes are not particularly limited, and known foreign genes can be adequately used depending on purposes. For example, biosynthesis genes for an anthocyanin pigment or a carotenoid pigment or genes for regulating pigment synthesis, genes for regulating morphogenesis (e.g., flowering or plant height), genes that confer disease resistance, genes that confer drought resistance, and the like are included.

As a vector for introducing a gene of interest into Agrobacterium or a microorganism capable of introducing a gene therein by an analogous mechanism to that of Agrobacterium, pBI121 (DDBJ Accession No. AF485783) or the like can be used. Furthermore, pKT11, an improved type of the above vector, (JP Patent Publication No. 2001-161373 A) can also be used. Furthermore, a vector to be used may contain, in addition to a gene of interest, other genes such as a reporter gene, a selection marker gene, and a promoter gene. Examples of a reporter gene include a luciferase gene, a GFP gene, and a GUS gene. As a luciferase gene, a luciferase gene containing introns can also be used (DDBJ Accession No. U84006). When such a luciferase gene is contained in Agrobacterium or a microorganism capable of introducing a gene therein by an analogous mechanism to that of Agrobacterium, any effect of the luciferase gene carried by Agrobacterium or microorganism remaining in plants can be eliminated, and only the expression of the luciferase gene in plants can be confirmed.

Examples of the selection marker gene include a kanamycin resistance gene, a G418 resistance gene, and a hygromycin resistance gene.

Furthermore, examples of the promoter gene include a Maize ubiquitin promoter [Plant Mol Biol. 1994; 26(3): 1007-12] and a cauliflower mosaic virus 35S promoter (JP Patent No. 2645217).

Adequate combination of known elements such as a translation enhancer and a terminator can be contained. Examples of a virus-derived translation enhancer include sequences of a tobacco mosaic virus, alfalfa mosaic virus RNA4, bromo mosaic virus RNA3, potato virus X, and a tobacco etch virus [Gallie et al., Nuc. Acids Res., 15 (1987) 8693-8711]. Moreover, examples of a plant-derived translation enhancer include, a soybean β-1,3 glucanase (Glu)-derived sequence [written by Isao Ishida and Norihiko Mitsusawa, edited by Kodansha Scientific, Introduction for Cell Engineering Experimental Protocols (Saibo Kogaku Jikken Sousa Nyumon), Kodansha (Tokyo, Japan), p.119, 1992] and a sequence from tobacco ferredoxin-binding subunit (PsaDb) [Yamamoto et al., J. Biol. Chem., 270 (1995) 12466-12470]. Examples of a terminator include an nos gene terminator and an ocs gene terminator [Annu. Rev. Plant Physiol. Plant Mol. Biol., 44 (1993) 985-994, "Plant Genetic Transformation and Gene Expression; A Laboratory Manual," edited by Draper, J. et al., Blackwell Scientific Publication, 1988]. Moreover, it has been reported that a plurality of 35S gene enhancer portions (which have been identified as transcriptional enhancers in promoters) can be aligned and ligated, so as to enhance activity (Plant Cell, 1 (1989) 141-150). Such portion can also be used as a portion of a DNA strand. These various elements are preferably incorporated into a DNA strand according to their properties, so that each of them can function. Such procedures can be appropriately performed by persons skilled in the art.

The above vectors can be easily produced by persons skilled in the art with the use of techniques that are generally used in the field of genetic engineering. Moreover, the vector of the present invention is not limited to a vector that is isolated from a natural source and may be an artificial construct, as long as it has the aforementioned structure. The DNA strand can be obtained by synthesis according to a method for nucleic acid synthesis, which is known and generally used.

The above vector in which a target gene and other genes have been incorporated can be introduced into Agrobacterium or a microorganism capable of introducing a gene therein by an analogous mechanism to that of Agrobacterium, by a technique known by persons skilled in the art, such as an electroporation method or a freeze-thaw method.

A solution that can be used herein as a solution for infection (medium for inoculation) is, but is not limited to, a solution that is prepared by diluting Agrobacterium or a microorganism capable of introducing a gene by an analogous mechanism to that of Agrobacterium, which has been precultured on liquid medium such as LB, YEP, or YMB and then collected by centrifugation, with the same medium, a medium for plant culture such as MS or B5, or the like.

The concentration of the aforementioned microorganism in a solution for infection is not particularly limited, as long as the introduction of a gene into plant tissue sections can be sufficiently performed at a concentration. Examples of such concentration can be 10,000 to 10,000,000 cells/mL solution for infection, for example. To ensure infection, plant bodies may be immersed in a solution containing the microorganism at a high concentration for a long time. However, this is not desirable since this significantly damages the plants.

Next, to ensure infection with Agrobacterium or a microorganism capable of introducing a gene by an analogous mechanism to that of Agrobacterium, tissue sections for which immersion treatment has been completed are cultured on medium for coculture. Any medium for coculture may be used herein, as long as it contains ingredients required for culturing plant tissue sections, such as a carbon source, a nitrogen source, inorganic salts, and a solidification agent. As the carbon source, sucrose, glucose, or the like can be used. As inorganic salts, MS inorganic salts, B5 inorganic salts, and the like can be used. As the solidification agent, agar, Gel-Rite, or the like can be used. Moreover, plant growth regulatory substances may also be added in order to promote the callus induction after coculture.

Concerning the time for coculture, coculture is performed for 1 to 14 days, and preferably 1 to 5 days, for example. The medium for coculture is preferably supplemented with acetosyringone, preferably at a concentration of 100 mM. The pH or temperature for coculture is not particularly limited, as long as it does not adversely affect plant tissue sections. For example, the pH preferably ranges from 5 to 8, and the temperature preferably ranges from 20°C to 28°C. Furthermore, light conditions preferably entail a dark place.

### (4) Selection of transformed embryogenic callus

Agrobacterium or a microorganism capable of introducing a gene by an analogous mechanism to that of Agrobacterium adhering to cocultivated embryogenic calli is eliminated. Subsequently, embryogenic calli into which the relevant gene has been introduced are selected using callus selection medium. For example, sterilization is performed by planting the aforementioned medium for coculture on medium containing an antibiotic such as cefotaxime.

The medium for callus selection that is used herein is prepared by adding an antibiotic for sterilization and a substance for selection of transformed cells to the medium for callus proliferation. Examples of such antibiotics for sterilization include cefotaxime, moxalactam, and meropenem. The concentration of an antibiotic for sterilization preferably ranges from 100 µg/ml to 300 µg/ml, for example, when the antibiotic is cefotaxime.

An antibiotic, a herbicide, or an amino acid analog can be used for selection of transformed cells. When an antibiotic is used, it is not particularly limited, as long as it is an antibiotic appropriate for a marker gene for the above selection. Hygromycin is preferred herein. The concentration of an antibiotic for selection of transformed cells preferably ranges from, when it is hygromycin, 3 µg/ml to 100 µg/ml, for example.

Furthermore, light conditions preferably entail a dark place.

Transformed embryogenic calli can be obtained by employing the above medium conditions and light conditions and culturing embryogenic calli under general culture conditions for plant cells at 20°C to 28°C for 7 days to 4 months, for example.

### (5) Induction of somatic embryo from transformed embryogenic callus

Transformed embryogenic calli are planted and then cultured on medium for inducing somatic embryos, so as to perform induction of somatic embryos. Medium to be used herein for inducing somatic embryos may be any medium, as long as it contains a carbon source, a nitrogen source, inorganic salts, and the like. As a carbon source, sucrose, glucose, or the like can be used, for example. As inorganic salts, an MS inorganic salt, a B5 inorganic salt, and the like can be used. Basic medium containing them can be used herein. Moreover, as a solidification agent, agar, Gel-Rite, or the like can be used. Induction can also be performed with the use of liquid medium. It is preferable to further add a plant growth regulatory substance.

Specifically, somatic embryo induction medium is prepared using MS medium or the like as basic medium that is supplemented with 0.5-4% , desirably 1- 2%, sucrose and 0.5-4%, desirably 0.5-2%, fructose as sugar sources. Furthermore, 1-6%, desirably 2-4%, sorbitol or mannitol may also be added as other sugars. Zero (0)-2 ppm, desirably 0.01-0.05 ppm, 2,4-dichlorophenoxyacetic acid (2,4-D) is used as a desirable auxin that is a plant growth regulatory substance; indole-3-acetic acid (IAA), indole-3-butylic acid (IBA), 1 -naphthalenacetic acid (NAA), 4-chlorophenoxyacetic acid (CPA), chloromethylphenoxyacetic acid (MCPA), 2,4,5-trichlorophenoxyacetic acid (2,4,5-T), dichloromethoxybenzoic acid (DICAMBA), and trichloroaminopicolinic acid (PICROLAM) are used as other auxins; 0-0.5 ppm, desirably 0.05-0.2 ppm, 6-benzyladenine (BA) is used as a desirable cytokinin; zeatin (ZEA), kinetin (KN), 6-(benzylamino)-9-(2-tetrahydropyranyl)-9H-purine (PBA), 2-isopentenyladenine (2ip), thidiazuron (TDZ), and the like are used as other cytokinins. Moreover, 1-10 mM, desirably 2-5 mM, glutamic acid and/or 1-10 mM, desirably 2-5 mM, proline is added. MES (0.1-10 mM) may also be added as a buffer. The pH of the medium ranges from 5 to 7. Regarding the light environment, light conditions (12-16 hours of light a day and photosynthetic photon flux density ranging from 5.7 µmole/m²/sec to 34.2 µmole/m²/sec) are desired, but dark conditions may also be employed herein. The temperature ranges from 20°C to 30°C and desirably ranges from 23°C to 27°C. The time for culture ranges from 4 to 12 weeks and desirably ranges from 5 to 10 weeks.

### (6) Germination and rooting of somatic embryo

Somatic embryos germinate on both solid medium and liquid medium with high efficiency. Medium to be used for germination is prepared using MS medium or the like as basic medium that is supplemented with 1-6%, desirably 2-4%, sucrose as a sugar source. When rooting takes place significantly to a greater extent than germination, 1-6%, desirably 2-4%, sorbitol or mannitol may also be added as another sugar to suppress such rooting. Addition of a plant growth regulatory substance is not particularly required, but auxins or cytokinins may be added to promote germination. The pH for the medium ranges from 5 to 7. Regarding light environment, light conditions of 12 hours to 16 hours of light a day and photosynthetic photon flux density ranging from 1.1 µmole/m²/sec to 34.2 µmole/m²/sec and desirably ranging from 2.3 µmole/m²/sec to 11.4 µmole/m²/sec are employed. The temperature ranges from 20°C to 30°C and desirably ranges from 23°C to 27°C. In the case of solid medium, the medium is solidified using agar (0.8-1.2%) or Gel-Rite (0.1-0.3%). The container is not particularly limited, as long as it is used for plant tissue culture. In the case of solid medium, the aforementioned plant box is used, and in the case of liquid medium, the aforementioned agitation-type or an air-lift-type fermenter is used. The time for culture ranges from 4 to 12 weeks in the case of solid medium and ranges from 3 to 6 weeks in the case of liquid medium. During such time for culture, somatic embryos take root and develop into plants in sizes that lead to the development of several leaves.

### (7) Transplantation in greenhouse

Plants regenarated from somatic embryos grow normally in a greenhouse. Soil to be used for transplantation is not particularly limited and may be commercially available culture soil for growth of seedlings. After transplantation of plants, it is desirable to perform appropriate humidification and light shielding for about 1 to 3 weeks.

### (8) Confirmation of gene transfer and gene expression in gene-introduced plant

Gene transfer and gene expression in calli or plants obtained by introducing genes with the use of the above procedures can be confirmed by a method using a reporter gene, which is known by persons skilled in the art, such as a method using luciferase luminescence, fluorescence of a green fluorescent protein (GFP), blue color development due to the use of β-glucuronidase (GUS), or the like. Moreover, according to a technique known by persons skilled in the art, such confirmation may also be performed by a PCR method, a Southern hybridization method, or a Northern hybridization method using primers prepared based on the sequence of the relevant gene.

Hereinafter, the present invention will be described in detail by examples as follows, but the scope of the present invention is not limited by these examples.

### EXAMPLES

### [Example 1] Obtaining Spathiphyllum embryogenic callus

Callus induction from different tissues of the culture seedlings of Spathiphyllum (cultivar: Petite) was performed, and thereafter from the thus induced calli were induced embryogenic calli.

The culture seedlings used in this Example had been maintained by subculture on solid medium (which was prepared by adding 3% sucrose and 0.8% agar (Wako Pure Chemical Industries.) to MS medium and then adjusted to pH 5.8) in the light (photosynthetic photon flux density 5.7 µmole/m²/sec and daylength 16 hours) at 25°C. As a culture container, the plant box (inner volume: 300 ml) produced by Asahi Techno Glass (Tokyo, Japan) was used.

Leaf sheaths were collected by peeling them off from cultured seedlings on week 4 of culture. Approximately 2-cm sections (leaf sheath bases) including peeled portions were used as explants. The explants were transferred to 6 sections/container containing MS medium (pH 5.8; hereinafter referred to as "callus induction and proliferation medium") supplemented with 3% sucrose, 4 ppm 2,4-D, 0.2 ppm BA, 100 ppm caseinate hydrolysate, and 5 mM MES, and then solidified with 0.8% agar (container, plant box; medium volume, 50 ml). Four (4) containers were used in total. The sections were cultured in the dark at 25°C for 8 weeks, and then the conditions of callus induction were studied.

As a result, in 92% (the number of sections from which calli had been induced/the total number of sections = 22/24) of cultured sections, calli were induced in the peeled portions. These calli were subcultured on the same medium at 0.2 g/container, and then cultured for 1 month, and subsequently embryogenic calli were selected. The embryogenic calli were induced at a rate of 50% (the number of containers in which embryogenic calli had been induced/the number of containers subjected to treatment = 4/8).

The embryogenic calli (0.05 g each) were transferred to a 500-ml Erlenmeyer flask in which 160 ml MS liquid medium (pH 5.8) containing 1% sucrose, 3% sorbitol, and 5 mM MES had been placed, followed by 6-weeks shake culture at 25°C in the light (daylength 16 hours and photosynthetic photon flux density 22.8 µmole/m²/sec) (80 rpm). Thus, somatic embryos were formed, which were then transferred at 0.2 g each to a plant box to which 50 ml MS medium (pH 5.8; hereinafter "germination medium") containing 3% sucrose and solidified with 0.8% agar had been added, followed by 8 weeks of culture at 25°C in the light (daylengh 16 hours and photosynthetic photon flux density 5.7 µmole/m²/sec). Thus, somatic embryos germinated so that complete plants were obtained. The embryogenic calli could be maintained for 2 years while retaining their ability to induce somatic embryos, by subculture on the callus induction and proliferation medium every 4 weeks.

Furthermore, in a similar manner to the techniques employed for the cultivar Petite, bases obtained by cutting off leaves from *in vitro* leaf sheaths of other Spathiphyllum cultivars, Double Take and Claudia (i.e., 70 base sections (Double Take) and 68 base sections (Claudia)) were transferred to the callus induction medium. Embryogenic calli could be induced by 4 to 8 months of culture at 25°C in the dark. Induction rate (%) ((the number of base sections from which embryogenic calli had been induced/the total number of base sections) × 100) were 80% and 88%, respectively; and the ratios (the number of base sections from which embryogenic calli had been induced/the total number of base sections) were 56/70 and 60/68, respectively. These embryogenic calli were proliferated on the callus induction and proliferation medium. In the following experiments, Double Take embryogenic calli having good greenhouse cultivation properties were used.

### [Example 2] Construction of plant transformation vector

A hygromycin resistance (hyg) gene was used as a selection marker, from a cauliflower mosaic virus 35S RNA promoter and an *Escherichia coli*-derived hyg gene in pIG121 (JP Patent Publication No. 11-69979 A (1999)). A Maize-derived ubiquitin promoter [Plant Mol Biol. 1994; 26 (3): 1007-12], a cauliflower mosaic virus 35S RNA promoter, and an agrobacteria-derived nos terminator were used from vector pBI121 (Clontech). These expression cassettes were substituted with a Gus gene and an NptII gene using pKT11, a binary-type vector capable of proliferating in Agrobacterium and *Escherichia coli,* as a basic vector, thereby constructing pKT 152 and pKT163 (Fig. 1)

### [Example 3] Preparation of gene-introduced agrobacteria

The vectors constructed in Example 2 were introduced into the *Agrobacterium tumefaciens* AGL0 strain (ATCC No. BAA-100) by an electroporation method (Plant Molecular Biology Manual, C2 (1994) 1-32 (Ed.) Gelvin and Schilperoort, Kluwer Academic Publishers).

### [Example 4] Infection of embryogenic callus with gene-introduced agrobacteria

The *Agrobacterium tumefaciens* AGL0 strain containing each vector was shake-cultured at 28°C for 12 hours in YEB liquid medium (5 g/l beef extract, 1g/l yeast extract, 5g/l peptone, 5 g/l sucrose , and 2 mM magnesium sulfate (pH 7.2)) containing 50 ppm kanamycin. One (1) ml of the culture solution was centrifuged at 10,000 rpm for 3 minutes to collect cells. The resulting cells was re-suspended in 10 ml minA medium (2.1 g/l dipotassium hydrogen phosphate, 0.9 g/l potassium dihydrogen phosphate, 0.2 g/l ammonium sulfate, 0.1 g/l sodium citrate dihydrate, 0.02% magnesium sulfate heptahydrate, and 0.2% glucose), thereby preparing a bacterial solution for infection.

One (1) g to 7 g of embryogenic callus obtained in Example 2 was immersed per transformation experiment (and in the case of 7 g, approximately 500 embryogenic callus masses each containing 20 to 50 embryogenic calli) in the above bacterial solution of Agrobacterium for 1 minute. Embryogenic calli were placed on sterile filter paper, so as to remove excess Agrobacterium. Subsequently, the embryogenic calli were transferred to a coculture medium (MS inorganic salt, 3% sucrose , 100 ppm caseinate hydrolysate, 4 ppm 2.4-D, 0.2 ppm BA, 5 mM MES, 100 mM acetosyringone, 0.8% agar, and pH 5.6) within Petri dishes, followed by 4 or 5 days of culture in the dark at 25°C.

### [Comparative Example 1] Selection upon induction of somatic embryo from embryogenic callus

Embryogenic calli in the coculture medium were transferred to a selection medium for somatic embryo induction (MS inorganic salt, 1% sucrose, 1% fructose, 3 mM proline, 3 mM glutamine, 0.2 ppm 2.4-D, 0.1 ppm BA, 5 mM MES, 6 ppm hygromycin, 250 ppm cefotaxime, 0.8% agar, and pH 5.6). In this experiment, it was verified whether or not transformants could be obtained.

Approximately 7 g of embryogenic callus obtained by the single experiment in Example 4 was transferred to the selection medium for somatic embryo induction. However, all embryogenic calli browned and died within 1 to 2 months after culture. Thus, transformed embryogenic calli and somatic embryos could not be obtained by this method. As a result, no transformed plants were obtained.

### [Example 5] Selection on medium for proliferation of embryogenic callus

Embryogenic calli infected with Agrobacterium (as obtained in Example 4) were transferred to a callus selection medium (MS inorganic salt, 3% sucrose, 100 ppm caseinate hydrolysate, 4 ppm 2.4-D, 0.2 ppm BA, 5 mM MES, 3 ppm or 6 ppm hygromycin, 250 ppm cefotaxime, 0.8% agar, and pH 5.6 luciferin solution: 5 mM luciferin and 0.1% triton X-100). Subculture was repeated while replacing with the fresh medium every month. Transformed calli were confirmed by measuring the activity of luciferase gene, which served as a marker gene.

The method employed in this Example comprises adding 5 µl to 10 µl of a luciferin solution to calli on the medium and then allowing the calli to stand for 15 minutes in the dark. Subsequently, photons emitted from calli were detected and subjected to quantity survey using ARGUS50 (produced by Hamamatsu Photonics (Shizuoka, Japan)) for 50 minutes, so that image analysis was performed.

The results of measuring luciferase activity on day 7 after culture on the callus selection medium are shown in Fig. 2. Luminescence resulting from the introduced luciferase gene could be observed in all the 10 arbitrarily selected embryogenic callus masses (which had been treated with the agrobacteria into which vector pKT152 or pKT165 had been introduced), which grew without undergoing browning but underwent. yellowing. It was revealed from this result that embryogenic calli had been efficiently infected with Agrobacterium and that the gene had been expressed in the embryogenic calli after the introduction of the luciferase gene.

Embryogenic callus masses were subcultured every month for 2 to 4 months and then transferred to medium for inducing somatic embryos. Fig. 3 shows the results of measuring luciferase activity 3 months after treatment with pKT 165-introduced Agrobacterium. Among the group showing good growth, 22 strongly emitting calli and 5 weakly emitting calli out of 42 calli could be confirmed. It was revealed that strongly emitting embryogenic calli were uniform, non-chimeric transformants.

### [Example 6] Regeneration of plants through somatic embryo formation from transformed embryogenic callus and germination

Transformed embryogenic calli obtained in Example 5 were transferred onto a medium for inducing somatic embryos (MS inorganic salt, 1% sucrose, 1% fructose, 3 mM proline, 3 mM glutamine, 0.2 ppm 2.4-D, 0.1 ppm BA, 5mM MES, 250 ppm cefotaxime, 0.8% agar, and pH 5.6). The transformed embryogenic calli were cultured in the light at 25°C. The calli greened during 1 to 2 months after culture and then formed somatic embryos.

Furthermore, the somatic embryos were transferred to a medium for regeneration of plants (MS inorganic salt, 1% sucrose, 0.8% agar, and pH5.6), thereby obtaining regenarated plants.

To confirm that the regenarated plants definitely contained the gene, the plants containing the luciferase gene as a foreign gene were detected from the regenarated plants by PCR. Thus, it was confirmed that the regenarated plants were transformants. Here, 5'-AGAGATACGCCCTGGTTCCT-3' (SEQ ID NO: 1) and 5'-ATAAATAACGCGCCCAACAC-3' (SEQ ID NO: 2) were used as primers for specific amplification of a unique sequence of the luciferase gene. PCR reaction conditions: after heating at 94°C for 5 minutes, 30 reaction cycles each consisting of 94°C (30 seconds), 55°C (1 minute), and 72°C (1 minute), followed by 10 minutes of final reaction at 72°C. In this reaction, Taq polymerase (TAKARA SHUZO (Kyoto, Japan)) was used as an enzyme. Reaction products were separeated by agarose electrophoresis, and were stained with ethidium bromide, then observed under ultraviolet rays.

With the use of the above techniques, as shown in Table 1, 33 Spathiphyllum plants in total could be obtained, which were different transformants obtained from approximately 21.9 g (fresh weight) of embryogenic calli into which the vector (pKT152 or pKT165) containing the gene had been separately introduced. Moreover, Fig. 4 shows the results of measuring luciferase activity in the leaf discs of regenarated plants for which gene introduction could be confirmed by PCR. Since luminescence could be confirmed on almost the entire surface of each of the emitting leaf discs, this revealed that the chimeric rate was very low.

**Table 1 Transformed Spathiphyllum**

| Experiment No. | Vector | Coculture period | Weight of embryogenic callus | Concentration of hygromycin | Selection period | Number of obtained transformants |
|---|---|---|---|---|---|---|
| 1 | pKT152 | 5 days | About 2.8 g | 3 ppm | 2 months | 3 |
| 2 | pKT152 | 5days | About 1.4 g | 3 ppm | 3 months | 3 |
| 3 | pKT165 | 5 days | About 1 g | 3 ppm | 2 months | 2 |
| 4 | pKT165 | 5 days | About 2 g | 3 ppm | 3 months | 7 |
| 5 | pKT165 | 5 days | About 1 g | 3 ppm | 4 months | 6 |
| 6 | pKT165 | 4 days | About 2 g | 3 ppm | 2 months | 3 |
| 7 | pKT165 | 4 days | About 7 g | 6 ppm | 2 months | 3 |
| 8 | pKT165 | 5 days | About 4.7 g | 6 ppm | 4 months | 6 |
| | | | | | Total | 33 |

### INDUSTRIAL APPLICABILITY

According to the present invention, easy introduction of a foreign gene into plants of the family Araceae is made possible. This enables the efficient production of transformed plants of the family Araceae and is industrially useful.

All publications, patents, and patent applications cited herein are incorporated herein by reference in their entirety.

## Claims

1. A method for transforming a plant of the family Araceae, comprising introducing a foreign gene into an embryogenic callus of the plant through infection of the callus with Agrobacterium or a microorganism capable of introducing a gene by an analogous mechanism to that of Agrobacterium, thereby preparing a transformed embryogenic callus.

2. The method of claim 1, wherein the embryogenic callus is from a leaf sheath as explant.

3. The method of claim 1, wherein the Araceae plant is of the genus *Spathiphyllum.*

4. The method of claim 1, further comprising inducing an somatic embryo from the transformed embryogenic callus, followed by germination and rooting, thereby producing a transformed plant.
